# EUROPÄISCHE PATENTANMELDUNG

(11) **EP 4 696 140 A1**
(43) Veröffentlichungstag der Anmeldung: **18.02.2026**
(21) Anmeldenummer: 25190676.4
(22) Anmeldetag: 21.07.2025
(51) Int. Cl.: A01N 59/00, A01N 31/14, A01N 57/18, A01N 33/08, A01P 1/00, A61L 2/18

(54) **KEIMREDUZIERENDE WÄSSRIGE ZUSAMMENSETZUNG UMFASSEND WASSERSTOFFPEROXID UND WIRKSAMKEITSSTEIGERNDE KOMPONENTEN**

(30) Priorität: 24.07.2024 DE 202024104157 U
(71) Anmelder: Knieler & Team GmbH, 21227 Bendestorf (DE)
(72) Erfinder: KNIELER, Roland, 21227 Bendestorf (DE)
(74) Vertreter: Müller Schupfner & Partner Patent- und Rechtsanwaltspartnerschaft mbB (Hamburg)

(57) **Zusammenfassung**

Gegenstand der vorliegenden Erfindung ist eine wässrige Zusammensetzung umfassend neben Wasser, Wasserstoffperoxid, Phenoxyethanol, Etidronsäure und eine Aminoverbindung mit einer Carbonsäure- und/oder Alkohol-Gruppe und die Verwendung der wässrigen Zusammensetzung zur Desinfektion, insbesondere harter Oberflächen.

## Beschreibung

Die vorliegende Erfindung betrifft eine wässrige Zusammensetzung umfassend neben Wasser, Wasserstoffperoxid, Phenoxyethanol, Etidronsäure und eine Aminoverbindung mit einer Carbonsäure- und/oder Alkohol-Gruppe und die Verwendung der wässrigen Zusammensetzung zur Desinfektion, insbesondere harter Oberflächen.

*Clostridioides difficile* ist ein anaerobes sporenbildendes Bakterium und ist häufig verantwortlich für das Auslösen von Durchfallerkrankungen. Ein neuer Stamm, Ribotyp 027, ist besonders virulent. Dies ist ein Grund dafür, dass der Bedarf an sporenwirksamen Desinfektionsmitteln, z.B. im Gesundheitswesen oder in der lebensmittelverarbeitenden Industrie, nach wie vor ungebrochen ist.

Sporen sind sehr schwer abzutöten. Sporenwirksame Desinfektionsmittel wie zum Beispiel Chlorbleichlauge oder Peressigsäure werden von Anwendern häufig als penetrant riechend und als reizend bewertet. Wasserstoffperoxid als Sauerstoffabspalter ist generell wirksam gegen bakterielle Sporen. Allerdings ist die Wirksamkeit für die praktische Anwendung entweder mit sehr hohen Konzentrationen von 7 Gewichtsprozent oder mehr Wasserstoffperoxid in Desinfektionsmittellösungen verbunden, oder aber die Desinfektion ist für eine praktische Anwendung nicht schnell genug. Desinfektionsmittel unterliegen heute in Europa einer strengen Regulierung. Sie fallen unter die regulatorische Kategorie der Biozide und dürfen für die Flächendesinfektion keine Einwirkzeiten von länger als eine Stunde aufweisen. Basis ist dabei für die Prüfung auf Sporenwirksamkeit die europäische Norm EN 17126, die hohe Prüfanforderungen stellt. Sie wurde 2019 veröffentlicht.

Etidronsäure wird neben anderen Stoffen als Stabilisator für Wasserstoffperoxid eingesetzt. Alternativ können auch andere Chelatbildner, wie zum Beispiel Ethylendiamintetraessigsäure (EDTA), als Stabilisatoren für Wasserstoffperoxid eingesetzt werden.

Es gibt sehr viele Ansätze die Wirksamkeit von Wasserstoffperoxid in Desinfektionsmittels zu erhöhen. EP 1987121 B1 offenbart die Kombination von Wasserstoffperoxid mit einer langkettigen Phosphorverbindung. Die EP 2329002 B1 lehrt mehrere Stoffe mit Wasserstoffperoxid zu kombinieren, darunter eine cyclische Carbonsäure und ein Blockcopolymer-Tensid. Die WO 2018150359 A1 offenbart Wasserstoffperoxid zusammen mit amphoteren Aminoxid-Tensiden einzusetzen. Die EP 2023733 B1 offenbart die Kombination von Wasserstoffperoxid und ethoxylierten Carbonsäuren. Die US 8304378 B2 offenbart die Kombination von Wasserstoffperoxid mit langkettigen Carbonsäuren, EP 1139762 B1 und EP 1473998 B1 haben die Kombination mit anionischen Tensiden zum Gegenstand. In der WO 2009/021336 A1 wird die Kombination von Wasserstoffperoxid und Glycolsäure mit Benzylalkohol und/oder Phenoxyethanol offenbart. Interessant ist hier das Beispiel 25 in Tabelle 9, das neben Wasserstoffperoxid auch 1-Hydroxyethyliden-bisphosphonsäure (Briquest ADPA-60W), Phenoxyethanol, ein anionisches Tensid, ein nichtionisches Tensid und einige weitere Stoffe enthält und einen pH von 2,7 hat und keinerlei Wirksamkeit zeigt. Erst die Zugabe von Glycolsäure statt Milchsäure (Beispiel 27) führt hier zu einer Wirksamkeit gegen Sporen.

Obwohl es also eine Vielzahl von Ansätzen gibt, die Wirksamkeit von Wasserstoffperoxid gegen Sporen zu verbessern, ist augenfällig, dass selbst in den Markt neu eingeführte Produkte auf Wasserstoffperoxid-Basis marktführender Teilnehmer bei der Einführung keine sporizide Wirksamkeit ausloben. Ein Beispiel hierfür ist das kürzlich eingeführte Produkt Incidin OxyWipe NG auf Basis von Wasserstoffperoxid und Phenoxyethanol des Unternehmens Ecolab, das in der Produktinformation auf die oben genannte EP 1987121 B1 verweist und keine sporizide Wirksamkeit beansprucht, die den Anforderungen der Norm EN 17126 - 2019-02 entspricht.

Es besteht also nach wie vor der Bedarf, die Wirksamkeit von Wasserstoffperoxid in wässrigen Zusammensetzungen so zu steigern, dass praxisgerechte, normenkonforme sporizide Desinfektionsmittel möglich werden. Dies ist die Aufgabe der vorliegenden Erfindung. Aufgabe der vorliegenden Erfindung ist es weiterhin, eine gebrauchsfähige, wässrige Desinfektionsmittelzusammensetzung zur Verfügung zu stellen, die sich zur Flächendesinfektion - insbesondere harter Oberflächen - eignet, geruchsarm ist und eine lange Haltbarkeit aufweist.

### Zusammenfassung der Erfindung

Die Aufgabe wird erfindungsgemäß gelöst mit der wässrigen Zusammensetzung gemäß Anspruch 1. Die wässrige Zusammensetzung ist keim reduzierend und kann als Desinfektionsmittelzusammensetzung eingesetzt werden. Bevorzugte Ausführungsformen sind nachfolgend erläutert oder Gegenstand der Unteransprüche.

Die Erfindung umfasst eine wässrige Zusammensetzung umfassend neben Wasser: Wasserstoffperoxid, Phenoxyethanol, Etidronsäure und mindestens eine der unten näher beschriebenen Aminoverbindungen. Es gilt für die Zusammensetzung in Bezug auf die Konzentration obiger Bestandteile der Zusammensetzung Folgendes, auch unabhängig voneinander in Bezug auf die Vorzugsbereiche, enthaltend:
- 0,5 Gew.-% bis 6,5 Gew.-%, insbesondere 1 Gew.-% bis 3 Gew.-%, Wasserstoffperoxid;
- 0,1 Gew.-% bis 4 Gew.-%, insbesondere 0,5 Gew.-% bis 3 Gew.-%, Phenoxyethanol;
- 0,01 Gew.-% bis 2 Gew.-%, insbesondere 0,05 Gew.-% bis 1,5 Gew.-%, Etidronsäure;
- 0,01 Gew.-% bis 2 Gew.-%, insbesondere 0,05 Gew.-% bis 1,5 Gew.-%, zumindest einer Aminoverbindung.

Das Gewichtsverhältnis von Etidronsäure zu der Aminoverbindung und wenn es mehrere der Aminoverbindungen (bzw. mehrere der bevorzugten Aminoverbindungen) sind, zu der Summe der Aminoverbindungen (bzw. der Summe der bevorzugten Aminoverbindungen) beträgt 10:1 bis 1:1, bevorzugt 5:1 bis 2:1.

Der Anteil des Wassers beträgt größer 70 Gew.-%.

Der pH-Wert der wässrigen Zusammensetzung beträgt 1 bis 4, bevorzugt 2 bis 3 (gemessen bei 25 °C).

Die zumindest eine Aminoverbindung ist eine nicht-cyclische organische Aminoverbindung mit zumindest einer Amino-Gruppe mit einer Molmasse von höchstens 500 g/mol.

Die Aminoverbindung weist
a) mindestens eine Hydroxy-Gruppe (-OH) auf;
   und/oder
b) mindestens eine Carboxy-Gruppe (-COOH) auf, wobei die Carboxy-Gruppe
   auch als Salz bzw. die Carboxy-Gruppen auch als Salze vorliegen können,
wobei
a) das Sauerstoff-Atom der Hydroxy-Gruppe durch mindestens ein Kohlenstoffatom von dem Stickstoff-Atom der Amino-Gruppe entfernt ist;
b) das Carbonyl-Kohlenstoffatom der Carboxy-Gruppe durch mindestens ein Kohlenstoffatom von dem Stickstoff-Atom der Amino-Gruppe entfernt ist.

Die längste zusammenhängende Kohlenstoffkette im Molekül der Aminoverbindung besteht aus bis zu drei zusammenhängenden Kohlenstoffatomen.

Die Angaben beziehen sich jeweils auf die Konzentration des jeweiligen Stoffes in der Zusammensetzung in Gew.-% und können durch Einwaage beim Zusammenbringen oder beispielsweise durch gängige Methoden wie Titration, HPLC oder GC-Analyse bestimmt werden.

Überraschend und nicht vorhersehbar war, dass obige wässrige Zusammensetzungen auf Basis von Wasser, Wasserstoffperoxid, Phenoxethanol und Etidronsäure eine normenkonforme sporizide Wirksamkeit aufweisen, wenn geringe Mengen der Aminoverbindung zugegeben sind und die wässrige Zusammensetzung den erforderlichen pH-Wert hat.

Ein weiterer Gegenstand der Erfindung ist die nicht-therapeutische Verwendung der wässrigen Zusammensetzung zur nicht-therapeutischen Desinfektion von harten Oberflächen. Unter harten Oberflächen sind beispielsweise Fußböden, Wände, Tische und Regale, Behälter oder Gerätschaften, wie sie beispielsweise in der Lebensmittel- und Agrarlebensmittelindustrie oder bei der Zubereitung, Verarbeitung und Verpackung von Lebensmitteln und Getränken, in der Bioindustrie, der pharmazeutischen Industrie, der Kosmetikindustrie, in Reinräumen, in Gewächshäusern und Pflanzen- oder Tierzuchtgebäuden oder auch auf dem Gebiet der Hygiene und Gesundheit verwendet werden.

Ein besonderes Anwendungsgebiet für die erfindungsgemäßen Zusammensetzungen ist die Desinfektion von Krankenhausräumen, Arztpraxen und Pflegeheimen. "Nicht-therapeutisch" bedeutet, dass es sich um keine Anwendung am menschlichen oder tierischen Körper handelt.

Die zu behandelnden Oberflächen können unterschiedlich sein, insbesondere harte Oberflächen; genannt seien beispielsweise gewerbliche oder öffentliche Einrichtungen, Fußböden, Gerätschaften, Werkzeuge und Utensilien, Behältnisse wie Tanks, konservierende Verpackungen, Rohre und Schläuche.

Die zu behandelnde Oberfläche kann aus einem beliebigen Material und insbesondere aus Glas, Aluminium oder rostfreiem Stahl, Keramik oder organischem Polymer, wie beispielsweise Polyethylen, Polypropylen, Polycarbonat, Polyamid, Polyester, wie Polyethylenterephthalat (PET), Polyurethan, Fluorpolymeren, wie PTFE, PVDF und PFA, und Polyvinylchlorid (PVC) bestehen.

Nach einer Ausgestaltung der vorliegenden Erfindung wird die wässrige Zusammensetzung von einem porösen Material, wie einem Tuch, Schwamm, Papier, Bürstenfasern oder Stoff, absorbiert, wobei man eine gebrauchsfertige Formulierung auf einem festen Träger, wie beispielsweise einem Vliestuch, Mopp oder einem anderen textilen Material, erhält. Die Tücher können z.B. aus Cellulose, Cellulosederivate, Viskose, PET, PE, und/oder PP sein. Die Mopps können auch mehrlagig sein, wobei die Lagen aus unterschiedlichen Materialien bestehen können.

Die Mopps und Tücher werden z.B. vorkonfektioniert, d.h. mit der wässrigen Zusammensetzung getränkt und sind in dieser Form z.B. in Kunststoffbeuteln oder - gefäßen für den Einsatz angeboten. Diese können auch für den Einmalgebrauch vorgesehen sein.

Die wässrige Zusammensetzung kann auch aufgesprüht oder auf jede andere Weise auf die Fläche aufgebracht werden und dann mit einem Tuch oder Mopp verteilt werden.

### Detaillierte Beschreibung der Erfindung

Die Aminoverbindung weist eine oder mehrereAmino-Gruppen auf und hat eine einer Molmasse von höchstens 500 g/mol.

Die Aminoverbindung beinhaltet eine oder mehrere Substrukturen der Formel NR₃₋ₓHₓ, wobei kovalente Bindungen zwischen dem Stickstoffatom und einem Kohlenstoffatom des Restes R vorliegen. X ist 0, 1 oder 2.

Im pH-Bereich der erfindungsgemäßen Formulierungen können die Amino-Gruppen auch alle oder teilweise protoniert sein.

Vorzugsweise nicht unter die Definition der erfindungsgemäß eingesetzten Aminoverbindungen fallen Verbindungen, die
- quartäre Ammonium-Gruppen mit einer Substruktur NR₄⁺, und/oder
- N-Oxide-Gruppen, und/oder
- Amid-Gruppen, und/oder
- Ester-Gruppen, und/oder
- Keto-Gruppen, und/oder
- Aldehyd-Gruppen
enthalten und weiter bevorzugt
- auch keine anderen funktionellen Gruppen, ausgenommen solche wie in Anspruch 1 beschrieben, haben, wie z.B. keine phosphor- oder schwefelbasierten funktionelle Gruppen.

Vorzugsweise enthalten die Aminoverbindungen nur H, C, O und N Atome. Diese Definition erstreckt sich nicht auf das Gegenion der Aminoverbindungen im Falle eines Salzes. Das Gegenion kann z.B. Na⁺, K⁺, Ca²⁺, Mg²⁺ oder NH₄⁺ sein.

Der Terminus, dass "die längste zusammenhängende Kohlenstoffkette im Molekül aus bis zu drei zusammenhängenden Kohlenstoffatomen besteht" bedeutet, dass z.B. langkettige Amine wie z.B. Laurylamin-Derivate nicht unter die Definition der Aminoverbindungen fallen.

Beispiele für erfindungsgemäß eingesetzte Aminoverbindungen sind z.B. Monoethanolamin, Glycin, Tetrahydroxypropylethylendiamin oder Methylglycindiessigsäure-Trinatriumsalz.

Bestandteil der wässrigen Zusammensetzung können weiterhin sein: ein oder mehrere anionische Tenside, insbesondere C10- bis C22- Fettalkoholsulfate, C10-bis C22- Fettalkoholethersulfate, C10- bis C22- Fettalkoholsulfonate und/oder C10- bis C22- Olefinsulfonate, vorzugsweise Natriumlaurylsulfat, Natriumlaurylethersulfat, Natriumcocosulfat, Alkylsulfonate, verzweigt und unverzweigt, und/oder alpha-Olefinsulfonate.

Bestandteil der wässrigen Zusammensetzung können weiterhin sein: ein oder mehrere nicht-ionische Tenside wie Alkoholalkoxylate und/oder Alkoholalkoxylatether mit unterschiedlicher Kettenlänge (C5- bis C22-) und einem Alkoxylierungsgrad von z.B. x=3-25 (z.B. (-CH₂-CH₂-O-)ₓ -Einheiten). In manchen Fällen sind Kombinationen von Alkoholethoxylaten mit höherer Kettenlänge (C >= 12, Fettalkoholalkoxylate) und Alkoholethoxylaten oder Alkoholalkoxylatethern niedrigerer Kettenlänge (C < 12) vorteilhaft, wobei der Gewichtsanteil der längerkettigen Fettalkoholethoxylate jeweils niedriger ist. Beispiele für Alkoholalkoxylate höherer Kettenlänge sind z. B. C₁₆-C₁₈ Alkoholethoxylat, 25 EO (z.B. Lutensol AT 25, BASF), Iso C₁₃ Alkoholethoxylat 8 EO (z.B. Lutensol TO89, BASF). Ein Beispiel für ein Alkoholalkoxylat niedriger Kettenlänge ist Hexan-1-ol ethoxyliert (z.B. Lutensol CS 6250, BASF). Beispiel für einen Alkoholalkoxylatether ist der modifizierte Alkohol Polyglykolether, 22 EO, CAS 501019-90-5 (z.B. Dehypon wet, BASF).

Bestandteil der wässrigen Zusammensetzung können weiterhin sein: ein Lösemittel wie C2-/C3-Alkohole, wie Ethanol, Propan-1-ol und Propan-2-ol und/oder ein weiterer Lösungsvermittler mit jeweils Ether- und/oder Alkohol- und/oder Ester-Gruppen, insbesondere Ether- und/oder Alkohol-Gruppe, wie Di- oder Polyole, weiter jeweils bevorzugt mit 3 oder 4 oder 5 zusammenhängenden Kohlenstoffatomen in zumindest einer der Gruppen (ggf. auch nur in einem Segment oder auch jeweils Segmenten von z.B. 3 plus 3 oder 3 plus 4 oder 4 plus 4 Kohlenstoffatomen) und vorzugsweise maximal 8 Kohlenstoffatomen pro Molekül. Die Lösungsmittel oder Lösungsvermittler werden vorzugsweise zu 1 bis kleiner 20 Gew.-%, bevorzugt 2 bis kleiner 15 Gew.-%, besonders bevorzugt 2 bis kleiner 10 Gew.-% eingesetzt.

Bestandteil der wässrigen Zusammensetzung können weiterhin sein: weitere Stoffe, die den pH-Wert regulieren wie anorganische Säuren, wie z.B. Phosphorsäure oder Schwefelsäure, anorganische Basen wie z.B. Natriumhydroxid, oder Kaliumhydroxid oder Kaliumcarbonat. Zusätzlich können weiterhin Korrosionsinhibitoren wie Phosphorsäureester oder Benzotriazol sowie Stoffe zur Geruchsverbesserung und Farbstoffe und/oder Duftstoffe und/oder je nach Einsatzbereich Entschäumer oder Schaumbildner und/oder Reinigungsverstärker enthalten sein.

Die wässrige Zusammensetzung besteht vorzugsweise aus den vorgenannten Komponenten bzw. Verbindungen zu größer 97 Gew.-%, bevorzugt größer 99 Gew.-%, besonders bevorzugt größer 99,9 Gew.-%.

Die wässrige Zusammensetzung kann auch zum Tränken von Tüchern zur Reinigung bzw. Desinfektion kleinerer Flächen und zum Tränken von Mopps zum Reinigen bzw. Desinfizieren größerer Flächen wie Fußböden verwendet werden. Bevorzugt werden diese Tücher und Mopps gebrauchsfertig vorkonfektioniert und verpackt, besonders bevorzugt gebrauchsfertig vorkonfektioniert und verpackt mit Tüchern oder Mopps auf Viskose-Basis, wie aus Viskose, z.B. hergestellt im Lösemittelverfahren (Lyocell) oder auch aus hydrophobierten Viskose-Varianten.

Hervorzuheben ist, dass sich die wässrige Zusammensetzung auch in oder auf Tüchern oder Mopps, z.B. auf jeweils Viskose-Basis, durch eine hohe Lagerstabilität auszeichnet.

### Experimenteller Teil

Der Gehalt von Wasserstoffperoxid und Phenoxyethanol liegt nach 18 Wochen Lagerung bei 30 °C noch in einem Bereich von +/-5% des ursprünglichen Werts, der pH-Wert bleibt im Wesentlichen stabil. Die Daten wurden nach Lagerung der vorgetränkten Tücher, verpackt in einem sogenannten Flowpack (Schlauchbeutelverpackung mit obenliegender Entnahmeöffnung), an der ausgepressten wässrigen Zusammensetzung bestimmt.

Drei erfindungsgemäße Rezepturen sind in Tabelle 3 dargestellt. Zur besseren Vergleichbarkeit waren die Konzentrationen der bioziden Wirkstoffe Wasserstoffperoxid und Phenoxyethanol mit jeweils 2 Gew.-% identisch, der Gehalt an

Etidronsäure war mit 0,38 Gew.-% identisch, der Gehalt aller anderen Inhaltsstoffe wie Tenside und Lösemittel war identisch, nur der Gehalt der erfindungsgemäßen Aminverbindungen variierte. Der pH-Wert war bei allen drei Rezepturen auf einen identischen Wert von pH=2,1 bei 25 °C eingestellt worden. Aqua purificata war ad. 100%.

**Tabelle 1: Erfindungsgemäße Rezepturen mit Ergebnissen der Prüfung nach EN 17126, C. difficile, 60 Minuten, Ergebnisse dargestellt in log-Reduktionen einer 80%-igen Konzentration und einer 97%-igen Konzentration**

| Konzentrationsangaben in Gew.-% | Beispiel 1 | Beispiel 2 | Beispiel 3 |
|---|---|---|---|
| H₂O₂ | 2 | 2 | 2 |
| Phenoxyethanol | 2 | 2 | 2 |
| Etidronsäure | 0,38 | 0,38 | 0,38 |
| Methylglycindiessigsäure-Trinatriumsalz | 0,1 | | |
| Tetrahydroxypropyl Ethylendiamin | | 0,1 | |
| Glycin | | | 0,06 |

| Rest jeweils gleich Tenside und Lösemittel und Wasser ad 100 Gew.-% | | | |
|---|---|---|---|
| pH-Wert | 2,1 | 2,1 | 2,1 |
| Ergebnisse EN 17126 | | | |
| 80%-Lösung, log-Reduktion | 4,74 | 5,4 | 4,7 |
| 97%-Lösung, log-Reduktion | 4,24 | 5,0 | 4,2 |

Die Prüfungen nach EN 17126 entsprachen den Normanforderungen und waren in einem akkreditierten Labor ermittelt worden. Die Konzentrationen von 80 Gew.-% und 97 Gew.-% waren technisch bedingt.

Eine ausreichende Wirksamkeit der geprüften Lösung ist erreicht, wenn eine Reduktion von 4 log-Stufen bei 97% erreicht ist. Man sieht, dass die erforderliche Wirksamkeit auch bei 80% Verdünnung erreicht wurde. Damit konnte eine sehr gute, normkonforme Wirksamkeit nach EN 17126 gegen *C. difficile* gezeigt werden.

**Tabelle 2: Weitere, erfindungsgemäße Beispiele 4 bis 11 und 14 bis 17 und nicht erfindungsgemäße Beispiele 12 und 13**

| Konzentrationsangaben in Gew.-% | Beispiel 4 | Beispiel 5 | Beispiel 6 | Beispiel 7 | Beispiel 8 |
|---|---|---|---|---|---|
| H₂O₂ | 2,4 | 2 | 3 | 1 | 0,5 |
| Phenoxyethanol | 2,4 | 2 | 0,5 | 3 | 3 |
| Etidronsäure | 0,38 | 0,38 | 0,4 | 1,2 | 0,06 |
| Monoethanolamin | | 0,05 | | | |
| Methylglycindiessigsäure-Trinatriumsalz | | | | 0,1 | 0,06 |
| Tetrahydroxypropylethylendiamin | | | 0,2 | | |
| Glycin | 0,06 | | | 0,15 | |
| Tensid 1 | | | 0,2 | | 0,2 |
| Tensid 2 | | | 0,2 | 0,1 | 0,2 |
| Tensid 3 | 0,4 | 0,5 | | | |
| Tensid 4 | | | | 0,2 | 0,2 |
| Tensid 5 | | | 0,2 | | |
| Tensid 6 | 0,045 | 0,045 | 0,02 | 0,02 | 0,02 |
| Lösemittel 1 | 9 | 8 | | 15 | 10 |
| Lösemittel 2 | | | 12 | | |
| Lösemittel3 | | | | | |
| Phosphorsäure/ Kaliumhydroxid | | | auf pH 2,2 | auf pH 2,2 | auf pH 2,2 |
| pH-Wert | 2 | 2,1 | 2,2 | 2,2 | 2,2 |

**Tabelle 2 (Fortsetzung)**

| **Konzentrationsangaben in Gew.-%** | Beispiel 9 | Beispiel 10 | Beispiel 11 | Beispiel 12 | Beispiel 13 |
|---|---|---|---|---|---|
| H₂O₂ | 2 | 2 | 2 | 2,4 | 2 |
| Phenoxyethanol | 2 | 2 | 2 | 2,4 | 2 |
| Etidronsäure | 0,4 | 0,4 | 0,4 | 0,15 | 0,5 |
| Monoethanolamin | 0,05 | 0,08 | 0,08 | | |
| Methylglycindiessigsäure-Trinatriumsalz | | | | | |
| Tetrahydroxypropylethylendiamin | | | | | |
| Glycin | | | | | |
| Tensid 1 | 0,2 | 0,2 | 0,2 | | 0,2 |
| Tensid 2 | 0,2 | 0,2 | 0,2 | | 0,2 |
| Tensid 3 | | | | 0,4 | |
| Tensid 4 | 0,2 | 0,2 | 0,2 | | |
| Tensid 5 | | | | | |
| Tensid 6 | 0,02 | 0,02 | 0,02 | 0,045 | 0,045 |
| Lösemittel 1 | 15 | 10 | | 9 | 8 |
| Lösemittel 2 | | | 8 | | |
| Lösemittel3 | | | | | |
| Phosphorsäure/ Kaliumhydroxid | auf pH 2,2 | auf pH 2,2 | auf pH 2,2 | | auf pH 2,2 |
| pH-Wert | 2,2 | 2,2 | 2,2 | 2,1 | 2,2 |

**Tabelle 2 (Fortsetzung)**

| **Konzentrationsangaben in Gew.-%** | Beispiel 14 | Beispiel 15 | Beispiel 16 | Beispiel 17 |
|---|---|---|---|---|
| H₂O₂ | 2 | 2 | 2 | 2 |
| Phenoxyethanol | 2 | 2 | 2 | 2 |
| Etidronsäure | 0,38 | 0,4 | 0,4 | 0,4 |
| Monoethanolamin | | | | |
| Methylglycindiessigsäure-Trinatriumsalz | 0,1 | 0,1 | 0,1 | 0,1 |
| Tetrahydroxypropylethylendiamin | | | | |
| Glycin | | | | |
| Tensid 1 | | 0,2 | 0,2 | 0,2 |
| Tensid 2 | | 0,2 | 0,2 | 0,2 |
| Tensid 3 | 0,4 | | | |
| Tensid 4 | | 0,2 | 0,2 | 0,2 |
| Tensid 5 | | | | |
| Tensid 6 | 0,045 | 0,02 | 0,02 | 0,02 |
| Lösemittel 1 | | 15 | 8 | |
| Lösemittel 2 | 8 | | | 8 |
| Lösemittel3 | | | 3 | |
| Phosphorsäure/ Kaliumhydroxid | | | | |
| pH-Wert | 2,1 | 2,1 | 2,1 | 2,1 |

| | | | | |
|---|---|---|---|---|
| Tensid 1: Sulfonsäuren, C14-16-alkan hydroxy und C14-C16-alken, Natriumsalze; Tensid 2: Natriumdodecylsulfat; Tensid 3: Sulfonsäuren, C14-17-sec-alkan, Natriumsalze; Tensid 4: Hexan-1-ol, ethoxyliert; Tensid 5: Modifizierter Alkoholpolyclycolether, 22EO; Tensid 6: Isotridecanol, ethoxyliert; Lösemittel 1: Ethanol; Lösemittel 2: Propan-1-ol; Lösemittel 3: 3-Methoxy-3-methylbutan-1-ol. | | | | |

## Patentansprüche

1. Wässrige Zusammensetzung enthaltend
0,5 Gew.-% bis 6,5 Gew.-%, insbesondere 1 Gew.-% bis 3 Gew.-%, Wasserstoffperoxid;
0,1 Gew.-% bis 4 Gew.-%, insbesondere 0,5 Gew.-% bis 3 Gew.-%, Phenoxyethanol;
0,01 Gew.-% bis 2 Gew.-%, insbesondere 0,05 Gew.-% bis 1,5 Gew.-%, Etidronsäure;
0,01 Gew.-% bis 2 Gew.-%, insbesondere 0,05 Gew.-% bis 1,0 Gew.-%, zumindest einer Aminoverbindung,
wobei die zumindest eine Aminoverbindung eine nicht-cyclische organische Aminoverbindung mit zumindest einer Amino-Gruppe und mit einer Molmasse von höchstens 500 g/mol ist,
wobei
die Aminoverbindung aufweist:
a) mindestens eine Hydroxy-Gruppe (-OH);
und/oder
b) mindestens eine Carboxy-Gruppe (-COOH), wobei die Carboxy-Gruppe/n auch als Salz/e vorliegen kann/können,
wobei
a) das Sauerstoffatom der Hydroxy-Gruppe durch mindestens ein Kohlenstoffatom von dem Stickstoff-Atom der Amino-Gruppe entfernt ist,
b) das Carbonyl-Kohlenstoffatom der Carboxy-Gruppe durch mindestens ein Kohlenstoffatom von dem Stickstoff-Atom der Amino-Gruppe entfernt ist,
wobei
die längste zusammenhängende Kohlenstoffkette im Molekül der Aminoverbindung aus bis zu drei zusammenhängenden Kohlenstoffatomen besteht; und wobei
- das Gewichtsverhältnis von Etidronsäure zu der Summe der Aminoverbindungen 10:1 bis 1:1, bevorzugt 5:1 bis 2:1, beträgt;
- der Anteil von Wasser mehr als 70 Gew.-% beträgt; und
- die wässrige Zusammensetzung einen pH-Wert von 1 bis 4, bevorzugt 2 bis 3, hat.

2. Die wässrige Zusammensetzung nach Anspruch 1 umfassend weiterhin ein oder mehrere anionische Tenside, insbesondere C10- bis C22- Fettalkoholsulfate, C10- bis C22- Fettalkoholethersulfate, C10- bis C22- Fettalkoholsulfonate und/oder C10- bis C22- Olefinsulfonate.

3. Die wässrige Zusammensetzung nach zumindest einem der vorhergehenden Ansprüche umfassend weiterhin ein oder mehrere nichtionische Tenside, insbesondere C5- bis C22-Alkoholalkoxylate und/oder Alkoholalkoxylatether, vorzugsweise mit einem Alkoxylierungsgrad von 3 bis 25.

4. Die wässrige Zusammensetzung nach zumindest einem der vorhergehenden Ansprüche umfassend weiterhin Ethanol und/oder Propan-1-ol und/oder Propan-2-ol, vorzugsweise insgesamt von 1 bis kleiner 20 Gew.-%, bevorzugt 2 bis kleiner 15 Gew.-%, besonders bevorzugt 2 bis kleiner 10 Gew.-%.

5. Die wässrige Zusammensetzung nach zumindest einem der vorhergehenden Ansprüche umfassend weiterhin mindestens einen Lösungsvermittler mit jeweils Ether- und/oder Alkohol- und/oder Ester-Gruppen, insbesondere Ether- und/oder Alkohol-Gruppe, wie Di- oder Polyole, weiter jeweils bevorzugt mit 3 oder 4 oder 5 zusammenhängenden Kohlenstoffatomen in zumindest einer der Gruppen, und vorzugsweise maximal 8 Kohlenstoffatomen pro Molekül, vorzugsweise insgesamt von 1 bis kleiner 20 Gew.-%, bevorzugt 2 bis kleiner 15 Gew.-%, besonders bevorzugt 2 bis kleiner 10 Gew.-%.

6. Die wässrige Zusammensetzung nach zumindest einem der vorhergehenden Ansprüche, wobei die Aminoverbindung Methylglycindiessigsäure-Trinatriumsalz und/oder Tetrahydroxypropylethylendiamin und/oder Glycin ist.

7. Die wässrige Zusammensetzung nach zumindest einem der vorhergehenden Ansprüche, wobei die Aminoverbindung Monoethanolamin ist oder umfasst.

8. Die wässrige Zusammensetzung nach zumindest einem der vorhergehenden Ansprüche enthaltend weiterhin als pH-Regulatoren anorganische Säuren oder Basen und/oder Korrosionsinhibitoren und/oder Duftstoffe und/oder Entschäumer oder Schaumbildner und/oder Reinigungsverstärker.

9. Die wässrige Zusammensetzung nach zumindest einem der vorhergehenden Ansprüche bestehend aus den in einem oder mehreren der vorhergehenden Ansprüche genannten Komponenten bzw. Verbindungen zu größer 97 Gew.-%, bevorzugt größer 99 Gew.-%, besonders bevorzugt größer 99,9 Gew.-%.

10. Tücher oder Mopps getränkt mit der wässrigen Zusammensetzung nach zumindest einem der vorhergehenden Ansprüche, bevorzugt gebrauchsfertig vorkonfektioniert und verpackt, wobei die Tücher oder Mopps bevorzugt jeweils Tücher oder Mopps auf Viskose-Basis sind.

11. Nicht-therapeutische Verwendung der wässrigen Zusammensetzung nach zumindest einem der Ansprüche 1 bis 9 oder der Tücher oder Mopps nach Anspruch 10 zur Desinfektion, insbesondere gegen *Clostridioides difficile* Sporen, wobei die Verwendung vorzugsweise *Clostridioides difficile* Sporen um zumindest 4log -Stufen in zumindest 1 Stunde gemäß EN 17126 reduziert.

12. Die nicht-therapeutische Verwendung nach Anspruch 11 zur Desinfektion harter Oberflächen.
